# EUROPÄISCHE PATENTSCHRIFT

(11) **EP 4 176 983 B1**
(45) Veröffentlichungstag und Bekanntmachung des Hinweises auf die Patenterteilung: **06.11.2024**
(21) Anmeldenummer: 22201332.8
(22) Anmeldetag: 13.10.2022
(51) Int. Cl.: B08B 9/027, B08B 9/032, B08B 13/00, G01N 21/31, G01N 21/3577, G01N 21/85, G01N 27/00, G01N 33/14, G01N 21/53, G01N 21/84, G05B 19/418

(54) **VERFAHREN ZUR PROZESSÜBERWACHUNG EINER ORTSGEBUNDENEN REINIGUNG EINER PRODUKTIONSEINHEIT ZUR HERSTELLUNG UND/ODER BEHANDLUNG FLÜSSIGER ABFÜLLPRODUKTE UND PRODUKTIONSANLAGE MIT EINEM REINIGUNGSSYSTEM ZUR ORTSGEBUNDENEN REINIGUNG**
METHOD FOR THE PROCESS MONITORING OF A LOCALIZED CLEANING OF A PRODUCTION UNIT FOR PRODUCING AND/OR TREATING LIQUID FILLING PRODUCTS
PROCÉDÉ DE SURVEILLANCE DE PROCESSUS D'UN NETTOYAGE LOCALISÉ D'UNE UNITÉ DE PRODUCTION POUR LA FABRICATION ET/OU LE TRAITEMENT DE PRODUITS DE REMPLISSAGE LIQUIDES ET INSTALLATION DE PRODUCTION DOTÉE D'UN SYSTÈME DE NETTOYAGE LOCAL

(30) Priorität: 04.11.2021 DE 102021128674
(43) Veröffentlichungstag der Anmeldung: 10.05.2023
(73) Patentinhaber: Krones AG, 93073 Neutraubling (DE)
(72) Erfinder: RAUCH, Matthias, 93073 Neutraubling (DE); HEIMRATH, Rohland, 93073 Neutraubling (DE); OEHMICHEN, Thomas, 93073 Neutraubling (DE)
(74) Vertreter: Grünecker Patent- und Rechtsanwälte PartG mbB

(56) Entgegenhaltungen:
- EP-B1- 2 362 008
- WO-A1-2021/209316
- DE-A1- 10 352 924
- US-A- 5 427 126

## Beschreibung

Die Erfindung betrifft ein Verfahren zur Prozessüberwachung einer ortsgebundenen Reinigung einer Produktionseinheit zur Herstellung und/oder Behandlung flüssiger Abfüllprodukte und eine Produktionsanlage mit einem Reinigungssystem zur ortsgebundenen Reinigung.

Ein Verfahren mit folgenden Merkmalen und eine entsprechende Produktionsanlage sind aus der WO 2021/209316 A1 bekannt: Eine Reinigungsflüssigkeit wird durch einen durch die Produktionseinheit führenden Reinigungskreislauf geleitet, wobei wenigstens ein für die Zusammensetzung der Reinigungsflüssigkeit charakteristischer Parameter im Vorlauf und Rücklauf des Reinigungskreislaufs im Vergleich zueinander gemessen wird. Dabei ermittelte Vorlaufwerte und Rücklaufwerte des charakteristischen Parameters werden durch den Reinigungskreislauf strömenden Volumenpaketen der Reinigungsflüssigkeit maschinell zugeordnet, und eine durch die Reinigung bedingte Veränderung der Reinigungsflüssigkeit wird durch Vergleich der Vorlaufwerte und Rücklaufwerte maschinell beurteilt.

Aus der EP 2 362 008 B1 ist es bekannt, in einer Produktionsanlage mit Produktionseinheiten zur Herstellung und/oder Behandlung flüssiger Abfüllprodukte eine Differenzmessung zwischen dem Vorlauf und Rücklauf eines Reinigungssystems zur ortsgebundenen Reinigung, auch Cleaning-In-Place (CIP) genannt, mittels spektroskopischer Verfahren beispielsweise in einer Umgehungsleitung der zu beurteilenden Hauptströmung einer Reinigungsflüssigkeit durchzuführen. Damit lässt sich die Zusammensetzung der Reinigungsflüssigkeit im Vergleich von Vorlauf und Rücklauf während eines Reinigungsprozesses abschätzen beispielsweise dahingehend, ob sich eine Wirckonzentration der Reinigungsflüssigkeit und/oder deren Schmutzfracht verändert hat.

Nachteilig dabei ist, dass zu vergleichende Vorlaufwerte und Rücklaufwerte im Wesentlichen gleichzeitig gemessen werden und daraus auf die Qualität der Reinigungsflüssigkeit geschlossen wird. Da die zu reinigenden Produktionsanlagen in der Regel vergleichsweise große Behälter- und Leitungsvolumina aufweisen, kann es zu erheblichen Ungenauigkeiten kommen betreffend die Aussagekraft der miteinander verglichenen Vorlaufwerte und Rücklaufwerte. Die Differenzen zwischen vorlaufseitigen und rücklaufseitigen Messwerten können dann auf bisher unerklärliche Weise schwanken. Beispielsweise können so vorübergehende Konzentrationserhöhungen in der Reinigungsflüssigkeit festgestellt werden und/oder stark schwankende Schmutzfrachten, was zu erwartenden Trends beim Ablauf von Reinigungsprogrammen oftmals widerspricht. Es ist dann schwierig, den Reinigungsfortschritt zu beurteilen, beispielsweise um geeignete Zeitpunkte für die Beendigung einzelner Programmschritte des Reinigungsprogramms festzulegen oder den Einsatz unterschiedlicher Reinigungsflüssigkeiten, wie Säure, Lauge und Wasser zu optimieren.

Es besteht daher Bedarf für eine präzisere Steuerung von Reinigungsprogrammen in derartigen Produktionsanlagen. Gewünscht ist zudem eine möglichst flexible Überwachung von den für die Zusammensetzung der Reinigungsflüssigkeit charakteristischen Parametern.

Die gestellte Aufgabe wird mit einem Verfahren und mit einer Produktionsanlage gemäß den unabhängigen Ansprüchen gelöst. Bevorzugte Ausführungsformen sind in den abhängigen Ansprüchen angegeben.

Demnach dient das Verfahren zur Prozessüberwachung einer ortsgebundenen Reinigung, auch CIP-Verfahren genannt, wenigstens einer Produktionseinheit zur Herstellung und/oder Behandlung flüssiger Abfüllprodukte, bei denen es sich beispielsweise um Getränke oder dergleichen flüssige Lebensmittel, pharmazeutische Produkte, Hygieneprodukte oder dergleichen handeln kann. Hierfür wird eine Reinigungsflüssigkeit durch einen durch die Produktionseinheit führenden Reinigungskreislauf geleitet und dabei wenigstens ein für die Zusammensetzung der Reinigungsflüssigkeit charakteristischer Parameter sowohl im Vorlauf als auch im Rücklauf des Reinigungskreislaufs im Vergleich zueinander gemessen.

Zudem werden dabei ermittelte Vorlaufwerte und Rücklaufwerte, also für den Vorlauf und Rücklauf jeweils ermittelte Werte des charakteristischen Parameters bestimmten durch den Reinigungskreislauf strömenden Volumenpaketen der Reinigungsflüssigkeit insbesondere jeweils paarweise maschinell zugeordnet. Ferner wird ein Fortschritt der Reinigung und/oder eine dadurch bedingte Veränderung der Reinigungsflüssigkeit durch Vergleich der den Volumenpaketen jeweils zugeordneten Vorlaufwerte und Rücklaufwerte maschinell beurteilt.

Unter einem Volumenpaket ist ein prinzipiell beliebig definierbares / abgrenzbares Teilvolumen der Reinigungsflüssigkeit zu verstehen, sofern man diesem zu einem bestimmten Zeitpunkt einen Aufenthaltsort innerhalb des Reinigungssystems zuordnen kann, beispielsweise einen bestimmten Leitungsabschnitt, einen Behälter oder eine Messstelle. Anders gesagt können Zeitpunkte bestimmt werden, zu denen sich derartige Volumenpakete / Teilvolumina an bestimmten Aufenthaltsorten befinden, beispielsweise im Bereich eines Messgeräts. Folglich kann man einzelnen durch das Reinigungssystem fließenden Volumenpaketen mit Messgeräten ermittelte Messwerte und gegebenenfalls zugehörige Messzeitpunkte zuordnen.

Folglich können Zeitpunkte von Vorlaufmessungen und zugehörigen Rücklaufmessungen zeitlich gezielt zueinander versetzt werden, im Gegensatz zur üblichen zeitgleichen Messung zu vergleichender Vorlaufwerte und Rücklaufwerte. Hierfür wird die Verweilzeit der Volumenpakete zwischen den zugehörigen Vorlaufmessungen und Rücklaufmessungen berücksichtigt. Anders gesagt wird mit der Rücklaufmessung jeweils gezielt solange gewartet, bis ein Volumenpaket, für das zuvor ein Vorlaufwert ermittelt wurde, am Messgerät zur Ermittlung des Rücklaufwerts ankommt. Es werden somit Vorlaufwerte und Rücklaufwerte desselben Volumenpakets miteinander verglichen.

Dadurch lassen sich prozessbedingte Schwankungen der charakteristischen Parameter besser beurteilen, beispielsweise eine sich verändernde Zusammensetzung der Reinigungsflüssigkeit im jeweiligen Vorratstank, ein Abbau reaktiver Inhaltsstoffe der Reinigungsflüssigkeit und/oder eine Zunahme der prozessbedingten Schmutzfracht. Somit lässt sich schließlich die Genauigkeit der vergleichenden Messung und deren Auswertung erhöhen, um den Fortschritt der Reinigung und/oder die dadurch bedingten Veränderungen der Reinigungsflüssigkeit präziser zu beurteilen. Folglich können einzelne Programmschritte der ortsgebundenen Reinigung präziser gesteuert werden, beispielsweise zur Vermeidung einer unzureichenden Reinigung und/oder einer unnötig langen Reinigung mit entsprechend hohen Medienverbräuchen.

Die beurteilte Zusammensetzung der Reinigungsflüssigkeit kann sich auf die Konzentration wenigstens eines Inhaltsstoffs / Wirkstoffs beziehen, auf eine Rezeptur / ein Mischungsverhältnis von Inhaltsstoffen / Wirkstoffen und/oder auf die jeweilige Schmutzfracht, also beispielsweise die Konzentration und/oder Art entfernter Verunreinigungen.

Unter der beschriebenen Reinigungsflüssigkeit sind sämtliche flüssige Medien zu verstehen, die zum Reinigen, Entkeimen und/oder Spülen der Produktionseinheit verwendet werden, beispielsweise Säuren, Laugen, Desinfektionslösungen und Spülwasser.

Die vergleichende Messung im Vorlauf und Rücklauf ist beispielsweise eine Differenzmessung der Vorlaufwerte und Rücklaufwerte ein und desselben Parameters.

Die Vorlaufwerte und Rücklaufwerte der Volumenpakete werden vorzugsweise an den jeweiligen Volumenpaketen direkt gemessen, könnten aber ersatzweise auch durch Interpolation und/oder Extrapolation von Vorlaufwerten und/oder Rücklaufwerten jeweils vorauseilender und/oder nachfolgender Volumenpakete ermittelt werden. Beispielsweise könnte der Vorlaufwert eines bestimmten Volumenpakets direkt gemessen werden und der damit zu vergleichende Rücklaufwert aus den Rücklaufwerten von dem bestimmten Volumenpaket vorauseilenden und nachfolgenden Volumenpaketen, oder umgekehrt. Auch in solchen Fällen ist eine Zuordnung von Vorlaufwerten und Rücklaufwerten zu Volumenpaketen im Sinne der Erfindung gegeben.

Charakteristische Parameter im Sinne der Erfindung können sowohl kontinuierlich im Hauptstrom der Reinigungsflüssigkeit gemessen werden, beispielsweise indem ein Messfühler ständig dem Hauptstrom ausgesetzt ist, und/oder diskontinuierlich an einer vom Hauptstrom abgezweigten Stichprobe der zu beurteilenden Reinigungsflüssigkeit, beispielsweise in einer Umgehungsleitung oder dergleichen Leitungsabzweig vom Hauptstrom.

Vorzugsweise wird wenigstens ein erster für die Zusammensetzung der Reinigungsflüssigkeit charakteristischer Parameter durch kontinuierliche Überwachung des Hauptstroms am jeweiligen Volumenpaket der Reinigungsflüssigkeit gemessen. Bei der kontinuierlichen Überwachung fließt das jeweilige Volumenpaket im Hauptstrom am zugeordneten Messgerät entlang. Darunter ist zu verstehen, dass der zugehörige Messfühler dem Hauptstrom ausgesetzt ist und der erste charakteristische Parameter daher prinzipiell durchgehend gemessen werden kann. Es versteht sich, dass die Messintervalle von Einzelmessungen hierfür geeignet angepasst werden können. Eine kontinuierliche Überwachung eignet sich besonders für erste charakteristische Parameter, die von der Strömung der Reinigungsflüssigkeit nur geringfügig oder nicht beeinflusst werden, beispielsweise von relativ starker und/oder turbulenter Strömung.

Der jeweilige erste charakteristische Parameter ist beispielsweise eine Leitfähigkeit, eine Trübung oder einen pH-Wert.

Vorzugsweise wird wenigstens ein zweiter für die Zusammensetzung der Reinigungsflüssigkeit charakteristischer Parameter an einer im jeweiligen Volumenpaket enthaltenen Stichprobe insbesondere mittels spektroskopischer Messung und/oder mittels Infrarot-Extinktionsmessung ermittelt und die Stichprobe dafür aus einem Hauptstrom der Reinigungsflüssigkeit abgezweigt. Die Stichprobe kann anschließend wieder in den Hauptstrom zurückgeführt werden. Eine derartige diskontinuierliche Messung an einer vom Hauptstrom abgezweigten Stichprobe ermöglicht eine Bestimmung des zweiten charakteristischen Parameters unter kontrollierten Strömungsverhältnissen und/oder über eine vorteilhafte Messdauer. Dies ist prinzipiell sowohl bei stillstehender Stichprobe als auch bei gleichmäßig und/oder relativ langsam strömender Stichprobe möglich.

Da dadurch strömungsbedingte Störungen des Messvorgangs vermieden und vergleichsweise lange Messdauern ermöglicht werden, können die diskontinuierlich erfassten Vorlaufwerte und Rücklaufwerte im Vergleich zu kontinuierlichen Messungen im Hauptstrom beispielsweise ein verbessertes Signal-Rausch-Verhältnis aufweisen. Zudem lassen sich an den abgezweigten Stichproben Messverfahren anwenden, die in unkontrolliert, stark und/oder turbulent strömender Reinigungsflüssigkeit nicht möglich oder praktikabel wären.

Die diskontinuierlichen Messungen an Stichproben der Reinigungsflüssigkeit könnten beispielsweise Messdauern für die einzelnen Stichproben von 0,1 Sekunden bis 5 Minuten aufweisen.

Vorzugsweise wird wenigstens ein erster für die Zusammensetzung der Reinigungsflüssigkeit charakteristischer Parameter kontinuierlich im Hauptstrom und wenigstens ein zweiter für die Zusammensetzung der Reinigungsflüssigkeit charakteristischer Parameter diskontinuierlich in einer vom Hauptstrom abgezweigten Stichprobe jeweils sowohl vorlaufseitig als auch rücklaufseitig gemessen. Durch Vergleich der daraus ermittelten Vorlaufwerte und Rücklaufwerte lassen sich für den jeweiligen Reinigungsprozess relevante Eigenschaften der Reinigungsflüssigkeit gezielt und gegebenenfalls umfassend beurteilen.

Prinzipiell wäre es auch denkbar, dass sich ein erster und zweiter charakteristischer Parameter nur durch die Art des verwendeten Messverfahrens unterscheiden in Sinne von kontinuierlich oder diskontinuierlich. Prinzipiell könnten daher durchaus Vorlaufwerte eines ersten Parameters mit Rücklaufwerten eines zweiten Parameters verglichen werden, oder umgekehrt, sofern sie dieselbe Messgröße betreffen, beispielsweise einen pH-Wert, und bestimmten Volumenpaketen im obigen Sinne zugeordnet sind. Auch wäre es denkbar, bestimmte charakteristische Parameter sowohl diskontinuierlich als auch kontinuierlich zu messen, beispielsweise im Rücklauf bestimmter Reinigungskreisläufe. Es könnten dadurch Plausibilitätskontrollen oder Reservemessungen ermöglicht werden.

Im Gegensatz zu lokalen Rückstandsmessungen, wie beispielsweise von Belägen an einzelnen Bestandteilen des Reinigungskreislaufs, ermöglicht die beschriebene kontinuierliche und/oder diskontinuierliche Messung des wenigstens einen charakteristischen Parameters eine integrale Beurteilung des Reinigungsfortschritts, um einen tatsächlichen Verschmutzungsgrad / eine Schmutzfracht in der jeweiligen Reinigungsflüssigkeit festzustellen und somit ein Maß für die Gesamtverunreinigung der betroffenen Produktionsanlage zu erhalten.

Durch kontinuierliche und/oder diskontinuierliche Messung der charakteristischen Parameter im Vorlauf lässt sich vor allem die initiale Zusammensetzung der jeweiligen Reinigungsflüssigkeit ermitteln / beurteilen.

In einem einzelnen Abzweig / in einer einzelnen Umgehung des Hauptstroms können wenigstens zwei unterschiedliche für die Zusammensetzung charakteristische Parameter gemessen werden.

Vorzugsweise wird die abgezweigte Stichprobe unabhängig vom Hauptstrom in den Bereich der zugeordneten diskontinuierlichen Messung gefördert und/oder vorder Messung gefiltert und/oder bei der Messung vorübergehend vom Hauptstrom abgetrennt und/oder nach der Messung wieder in diesen zurückgeleitet. Hierzu kann beispielsweise eine separat steuerbare Pumpe in einer den Hauptstrom umgehenden Umgehungsleitung angeordnet sein. Dies ermöglicht eine gleichmäßige und an die jeweilige Messung angepasste Förderung der Stichprobe durch den Bereich des zugeordneten Messgeräts. Optional kann vor diesem und/oder vor der Pumpe ein Filter angeordnet werden, mit dem die Messung störende Feststoffe zurückgehalten werden können. Eine strömungstechnische Abtrennung der Stichprobe vom Hauptstrom ist durch eingangsseitige und/oder ausgangsseitige Ventile am Abzweig / an der Umgehungsleitung möglich. Somit lassen sich für die jeweilige diskontinuierliche Messung an der Stichprobe reproduzierbare und an das jeweilige Messverfahren angepasste Messbedingungen herstellen.

Bei einer vorteilhaften Ausführungsform wird die Reinigungsflüssigkeit durch parallel geschaltete Reinigungskreisläufe geleitet, wobei für diese separat jeweils Vorlaufwerte und Rücklaufwerte ermittelt werden, die dann durch die einzelnen Reinigungskreisläufe geleiteten Volumenpaketen insbesondere paarweise zugeordnet und miteinander verglichen werden. Reinigungsfortschritte lassen sich dann für jeden einzelnen Reinigungskreislauf separat beurteilen, beispielsweise falls die Reinigungsflüssigkeit in den einzelnen Reinigungskreisläufen unterschiedliche Mengen an Schmutzfracht aufgenommen hat und/oder sich enthaltene Wirkstoffe unterschiedlich stark durch die dortige Reinigung verbraucht haben.

Bei dem Verfahren werden ferner Verweildauern einzelner Volumenpakete zwischen vorlaufseitigen und zugehörigen rücklaufseitigen Messungen sowohl unter Berücksichtigung des Fassungsvermögens dazwischenliegender Abschnitte des Reinigungskreislaufs und/oder zugehöriger Füllstände als auch auf der Grundlage einer Volumenstrommessung im Hauptstrom der Reinigungsflüssigkeit ermittelt. Die Volumenstrommessung ist beispielsweise im Vorlauf eines Reinigungskreislaufs besonders praktikabel. Durch Berücksichtigung des ermittelten Volumenstroms in Kombination mit betroffenen Fassungsvermögen und/oder Füllständen können prinzipiell Aufenthaltsorte und Aufenthaltszeitpunkte einzelner Volumenpakete im Reinigungskreislauf insbesondere zwischen vorlaufseitiger und zugehöriger rücklaufseitiger Messung ermittelt werden.

Durch zeitlich daran angepasste Steuerung der vorlaufseitigen und rücklaufseitigen Messungen können diese an identischen Volumenpaketen vorgenommen werden. Ebenso ist es möglich, einzelnen Vorlaufmessungen und/oder Rücklaufmessungen auf der Grundlage der berechneten Verweildauer einzelne Volumenpakete durch Interpolation oder Extrapolation unter Berücksichtigung jeweils vorauseilender und/oder nachfolgender Volumenpakete zuzuordnen.

Das Fassungsvermögen des jeweiligen Reinigungskreislaufs setzt sich aus den bei der ortsgebundenen Reinigung flüssigkeitsgefüllten Anlagenteilen zusammen, wie beispielsweise Rohrleitungen, Behältern, Wärmetauschern oder dergleichen. Sind Behälter nicht vollständig von der Reinigungsflüssigkeit gefüllt, so wird anstelle des jeweiligen Fassungsvermögens des Behälters der bei der Reinigung eingestellte Füllstand für die Berechnung der Verweildauer verwendet. Die beschriebene Zuordnung von Vorlaufwerten, Rücklaufwerten und Volumenpaketen zueinander basiert somit auf einer örtlichen und zeitlichen Verfolgung einzelner Volumenpakete durch den zu beurteilenden Reinigungskreislauf.

Anhand der Volumenstrommessung kann das in den jeweiligen Reinigungskreislauf einströmende Volumen der Reinigungsflüssigkeit ermittelt werden, beispielsweise durch kontinuierliche Volumenzählung. Eine kontinuierliche Volumenzählung ist beispielsweise mit einem Datenbaustein des Steuerungssystems möglich oder mit einem Register, welches durch die Messwerte eines Volumenzählers weitergeschaltet wird.

Vorzugsweise wird für den Rücklaufwert ein absoluter oder ein auf den zugehörigen Vorlaufwert bezogener relativer Schwellenwert vorgegeben und durch dessen Erreichen ein zugeordneter Reinigungsfortschritt maschinell festgestellt.

Beispielsweise kann ein Referenzwert, Referenzwertbereich und/oder mehrere Referenzwerte für einen oder mehrere für die Zusammensetzung der Reinigungsflüssigkeit charakteristische Parameter für den Rücklauf des Reinigungskreislaufs festgelegt werden, um ein Maß für eine ausreichend gereinigte Produktionsanlage und/oder einen entsprechend gereinigten Reinigungskreislauf vorzugeben. Erreicht der jeweilige gemessene Rücklaufwert dann diesen Referenzwert / Referenzwertbereich, so kann beispielsweise auf eine ausreichend gereinigte Produktionsanlage / einen entsprechend gereinigten Reinigungskreislauf geschlossen werden. Ebenso wäre ein entsprechender Vergleich zwischen Vorlaufwert und Rücklaufwert im Sinne einer vorgegebenen Differenz als Referenzwert / Referenzwertbereich denkbar.

Ebenso können derartige Referenzwerte auf der Grundlage gemessener Rücklaufwerte festgelegt werden, beispielsweise in historischem Vergleich mit entsprechenden Ergebnissen der ortsgebundenen Reinigung. Beispielsweise kann ein Referenzwert für einen ausreichend gereinigten Reinigungskreislauf festgelegt werden, indem eine ortsgebundene Reinigung wenigstens einmal und insbesondere mehrmals an einer ausreichend gereinigten bzw. entsprechend sauberen Produktionsanlage durchgeführt wird. Ergänzend oder alternativ kann ein Referenzwert ermittelt werden, indem eine ortsgebundene Reinigung wenigstens fünfmal oder wenigstens zehnmal durchgeführt wird und der dabei gemessene Rücklaufwert statistisch ausgewertet wird, beispielsweise als Mittelwert mit Standardabweichung. Ebenso ist es denkbar, entsprechende Referenzwerte historisch durch Vergleich mit weiteren Reinigungsprozessen anzupassen.

Bei Differenzmessungen kann beispielsweise bei Abnahme einer absoluten Differenz zwischen Vorlaufwert und Rücklaufwert auf einen bestimmten Schwellenwert davon ausgegangen werden, dass ein bestimmter Wirkstoff der Reinigungsflüssigkeit keine nennenswerte Reinigungswirkung mehr erzielt und der laufende Reinigungsprozess unterbrochen oder beendet werden kann.

Vorzugsweise wird ein Steuerungsprogramm für die ortsgebundene Reinigung (Reinigungsprogramm) und/oder ein Steuerungsprogramm für einen Produktionsbetrieb (Produktionsprogramm) der Produktionseinheit mittels eines Vergleichs der jeweils einander zugeordneten Vorlaufwerte und Rücklaufwerte mehrerer Volumenpakete automatisch angepasst. Beispielsweise kann durch eine maschinelle Analyse des zeitlichen Verlaufs von ermittelten Vorlaufwerten und Rücklaufwerten während des Reinigens (und Spülens) des Reinigungskreislaufs / der Produktionsanlage auf einen Reinigungsfortschritt / -erfolg des laufenden Reinigungsprogramms geschlossen werden.

Steuerungsprogramme für den Produktionsbetrieb können beispielsweise aufgrund maschineller Analyse von Vorlaufwerten und Rücklaufwerten angepasst werden, die beim Ausschieben von Abfüllprodukt gewonnen werden, um dadurch Rückschlüsse auf das Abfüllprodukt zu erhalten. Beispielsweise ist auf dieser Grundlage eine entsprechend angepasste Regelung von Sekundärkreisläufen beteiligter Wärmetauscher möglich. Ebenso lassen sich auf dieser Grundlage Qualität und Quantität von Ablagerungen beurteilen, so dass aus diesen wiederum Rückschlüsse auf den jeweiligen Produktionsbetrieb gezogen werden können. Dies ist insbesondere an davon besonders betroffenen Produktionseinheiten / Anlagenteilen möglich, wie Kurzzeiterhitzungsanlagen. Auch könnten beispielsweise Anzeichen für Defekte beispielsweise an Dichtungen festgestellt werden, indem eine Fracht zugehöriger Bestandteile / Materialien in der Reinigungsflüssigkeit überwacht wird.

Ergänzend zu den beschriebenen Messungen von Vorlaufwerten und Rücklaufwerten für die Zusammensetzung der Reinigungsflüssigkeit charakteristischen Parameter können von der Zusammensetzung im Wesentlichen unabhängige Anlagenparameter und/oder Behandlungsparameter wie beispielsweise die jeweilige Flüssigkeitstemperatur und/oder Volumenströme gemessen und/oder variiert werden, um einzelne Reinigungsprogramme der ortsgebundenen Reinigung zu optimieren. Beispielsweise könnte ein bestimmtes Reinigungsprogramm hinsichtlich mehrerer für die Zusammensetzung charakteristischer Parameter und/oder davon unabhängiger Parameter (siehe oben) in bestimmten Grenzen variiert werden, also beispielsweise hinsichtlich unterschiedlicher Temperaturen, Volumenströme und/oder Konzentrationen von Wirkstoffen der Reinigungsflüssigkeit.

Derartige Optimierungsprozesse könnten unter Zuhilfenahme künstlicher Intelligenz, also auf der Grundlage derartiger Algorithmen, zusätzlich selbstlernend optimiert werden.

Zusätzlich könnten Aromaverschleppungen beispielsweise in Nachspülwasser ermittelt werden, insbesondere, um eine Freigabe der Produktionseinheit für nachfolgenden Produktionsbetrieb auszulösen.

Bei einer weiteren günstigen Ausführungsform werden die Vorlaufwerte und Rücklaufwerte ferner einem bei der ortsgebundenen Reinigung der Produktionseinheit abgelaufenen Steuerungsprogramm und wenigstens einem der folgenden davon verwendeten Reinigungsparameter maschinell zugeordnet: Typen von Reinigungsflüssigkeiten; Grenzwerte für Leitwert und/oder Temperatur; Volumenströme der Reinigungsflüssigkeiten; Reinigungsdauer; Störungsmeldungen; Zeitstempel für Reinigungsbeginn und Reinigungsende; und Medienverbräuche. Dadurch lassen sich die Vorlaufwerte und Rücklaufwerte in einen für die Qualität von Reinigungsprozessen und/oder anschließenden Produktionsprozessen ausschlaggebenden Gesamtkontext stellen.

Beispielsweise lässt sich dokumentieren, welche Produktionseinheiten / Maschinen gereinigt werden, welche Reinigungsflüssigkeiten dafür benutzt wurden, also beispielsweise Lauge, Säure, Desinfektionsmittel oder dergleichen, und welche Qualität die jeweilige Reinigungsflüssigkeit aufwies. Dies kann beispielsweise einen Istwert der Leitfähigkeit betreffen, einen Grenzwert der Leitfähigkeit im Sinne eines Alarmparameters, bei dem die Reinigungszeit pausiert, einen Istwert der Temperatur, einen Grenzwert der Temperatur, der ebenso als Alarmparameter zu verstehen ist, einen zugehörigen Volumenstrom und/oder eine zugehörige Reinigungsdauer.

Ebenso könnten in diesem Sinne dokumentiert und berücksichtigt werden: die Identifikation des Reinigungsprogramms, also beispielsweise dessen Name und/oder Nummer; Störungsfreiheit bzw. Störungsmeldungen; Zeitstempel für das Reinigungsprogramm insgesamt und gegebenenfalls der Netto-Reinigungsdauer ohne prozessinterne Vorbereitungs- und/oder Wartezeiten für Tankvorbereitung oder dergleichen; Medienverbräuche insgesamt und spezifisch für einzelne Programmschritte.

Bei einer weiteren günstigen Ausführungsform werden wenigstens zwei der zuvor genannten Reinigungsparameter und/oder die einander zugeordneten Vorlauf- und Rücklaufwerte spezifisch für die Produktionseinheit und das aufgelaufene Steuerungsprogramm historisch miteinander verglichen und in eine maschinelle Analyse eines Heizverhaltens der Produktionseinheit, der Medienverbräuche der Reinigung, der Konzentration von Reinigungsflüssigkeiten und/oder der Zeiten für deren Kreislauf und Wechsel einbezogen. Das heißt, Parameter und Ergebnisse eines bestimmten Reinigungsprogramms können über einen historisch auswertbaren Anwendungszeitraum für eine bestimmte Produktionsanlage miteinander verglichen werden. Daraus ergeben sich beispielsweise folgende Möglichkeiten:
- Analyse des Heizsystems in Form einer Betrachtung des Temperaturgradienten beim Aufheizen eines Tanks, beispielsweise dahingehend, ob sich das Erwärmen um eine bestimmte Temperaturdifferenz historisch verlängert oder verkürzt hat, hinsichtlich möglicher Rückschlüsse auf einen geringeren Dampfdruck, einen Verschleiß von Regelventilen, eine Verunreinigung in Dampfleitungen und/oder Änderungen an Dampf-Druckminderern.
- Analyse der Medienverbräuche eines Reinigungsprogramms an einer bestimmten Produktionsanlage / Produktionseinheit, um beispielsweise auf Undichtigkeiten in einzelnen Produktionseinheiten auf der Grundlage gestiegener Verbräuche zu schließen.
- Analyse der Qualität einzelner Konzentrate, um beispielsweise zu bestimmen, wie viel Reinigungskonzentrat zum Anschärfen eines Tanks um 1 mS/cm benötigt wird. Dies kann beispielsweise hilfreich sein, um anwenderseitig Qualitätsschwankungen an Konzentraten von Reinigungsflüssigkeiten festzustellen.
- Zeitliche Analyse des Flüssigkeitskreislaufs und von Medienwechseln für bestimmte Reinigungsprogramme an einer bestimmten Produktionseinheit / Produktionsanlage, um beispielsweise tendenzielle Zunahmen von Zeitverlusten für die ortsgebundene Reinigung festzustellen.

Die beschriebene Produktionsanlage umfasst wenigstens eine Produktionseinheit zur Herstellung und/oder Behandlung flüssiger Abfüllprodukte, wie beispielsweise Getränke, und ein Reinigungssystem zur ortsgebundenen Reinigung der Produktionseinheit. Das Reinigungssystem umfasst wenigstens einen durch die Produktionseinheit führenden Reinigungskreislauf zum Durchleiten einer Reinigungsflüssigkeit und ein Überwachungssystem mit einer Auswerteeinheit und zugeordneten Messgeräten zur Vergleichsmessung wenigstens eines für die Zusammensetzung der Reinigungsflüssigkeit charakteristischen Parameters sowohl im Vorlauf als auch im Rücklauf des Reinigungskreislaufs.

Zudem sind im Reinigungskreislauf vorlaufseitige und rücklaufseitige Durchflussmesser angeordnet, und die Auswerteeinheit ist eingerichtet, sowohl mit den Messgeräten ermittelte Vorlaufwerte und Rücklaufwerte des Parameters wenigstens einem bestimmten durch den Reinigungskreislauf strömenden Volumenpaket der Reinigungsflüssigkeit gemeinsam zuzuordnen und einen Fortschritt der Reinigung und/oder eine dadurch bedingte Veränderung der Reinigungsflüssigkeit durch Vergleich der Vorlaufwerte und Rücklaufwerte des Volumenpakets zu beurteilen, als auch Verweildauern einzelner Volumenpakete zwischen vorlaufseitigen und zugehörigen rücklaufseitigen Messungen sowohl unter Berücksichtigung des Fassungsvermögens dazwischenliegender Abschnitte des Reinigungskreislaufs und/oder zugehöriger Füllstände als auch auf der Grundlage einer Volumenstrommessung im Hauptstrom der Reinigungsflüssigkeit zu ermitteln. Damit lassen sich die bezüglich des Anspruchs 1 beschriebenen Vorteile erzielen.

Vorzugsweise ist wenigstens eines der Messgeräte zur kontinuierlichen Überwachung einer Leitfähigkeit, einer Trübung und/oder eines pH-Werts im Hauptstrom der Reinigungsflüssigkeit ausgebildet. Das heißt, zugeordnete Sensoren sind im Wesentlichen kontinuierlich während der Messung dem Hauptstrom ausgesetzt. Zugehörige Messintervalle des Messgeräts für/zwischen Einzelmessungen können hierbei auf prinzipiell bekannte Weise im Sinne einer kontinuierlichen Überwachung angepasst werden.

Vorzugsweise ist ferner wenigstens eines der Messgeräte in einer den Hauptstrom der Reinigungsflüssigkeit umgehenden Umgehungsleitung zum Abzweigen einer Stichprobe aus der Reinigungsflüssigkeit angeordnet und zur qualitativen und/oder quantitativen Bestimmung von Bestandteilen der Reinigungsflüssigkeit insbesondere mittels spektroskopischer Messung und/oder Infrarot-Extinktionsmessung ausgebildet.

Ein solches Messgerät in der Umgehungsleitung (Bypass) ist zum Erfassen wenigstens eines Sensorsignals in einer vom Hauptstrom getrennten Stichprobe eingerichtet. Abhängig davon, welcher charakteristische Parameter der Reinigungsflüssigkeit zu bestimmen ist, kann das jeweilige Messgerät unterschiedliche Sensorik umfassen. Das jeweils im Sinne eines Vorlaufwerts oder Rücklaufwerts erfasste Sensorsignal kann beispielsweise eine Strahlungs-Extinktion durch Wechselwirkung mit der Stichprobe sein, insbesondere eine Infrarot-Extinktion zwischen Sensor und Empfänger für Infrarotlicht. Eine Infrarot-Extinktion ist beispielsweise indikativ für organische Bestandteile einer Schmutzfracht der Reinigungsflüssigkeit, da mit Infrarotlicht bekanntermaßen unterschiedliche molekulare Zustandspotenziale organischer Moleküle angeregt und damit gemessen werden können. Entsprechende Messungen sind insbesondere auch spektroskopisch möglich, also beispielsweise durch wellenlängenspezifische Extinktionsmessung entweder bei ausgewählten Wellenlängen oder über wenigstens einen vorgegebenen Wellenlängenbereich.

Durch derartige optische Messverfahren kann die chemische Zusammensetzung von Reinigungsflüssigkeiten gegebenenfalls sowohl qualitativ als auch quantitativ beurteilt werden. Beispielsweise können auf diese Weise unterschiedliche Stoffklassen bestimmt werden, wie beispielsweise Proteine, Stärke, Fette, und zwar gegebenenfalls sowohl qualitativ als auch hinsichtlich ihrer jeweiligen Konzentrationen. Als weitere Bestandteile der Reinigungsflüssigkeit können auch gelöste Ionen, wie beispielsweise Magnesium oder Kalzium bestimmt werden, ein bestimmtes in der Reinigungsflüssigkeit gelöstes Reinigungsmittel und/oder eine bestimmte Schmutzfracht, unter der im Wesentlichen die von der zu reinigenden Produktionseinheit abgelösten Verunreinigungen zu verstehen sind.

Die Umgehungsleitung (Bypass) ist als ein parallel zum Hauptstrom geschaltetes und hiervon vorzugsweise abtrennbares Teilstück des Reinigungskreislaufs zu verstehen. Die Umgehungsleitung umfasst einen Zulauf von einem den Hauptstrom führenden Hauptströmungsrohr und einen Ablauf zurück in das Hauptströmungsrohr.

Prinzipiell kann anstelle der Umgehungsleitung ein bezüglich der diskontinuierlichen Messung funktional gleichwertiger Leitungsabzweig aus dem Hauptstrom / dem Hauptströmungsrohr ohne Rückführung dorthin vorhanden sein. Das heißt, je nach der Zusammensetzung der Reinigungsflüssigkeit und/oder dem Volumen der abgezweigten Stichprobe könnte diese nach der Messung auch anderweitig abgeführt werden, beispielsweise in einen Behälter oder ein Abwassersystem.

In der Umgehungsleitung / im Leitungsabzweig kann wenigstens ein Filter angeordnet sein, um beispielsweise ein bestimmtes Messverfahren störende Feststoffe abzutrennen. Der Filter kann beispielsweise direkt am Zulauf der Umgehungsleitung / des Leitungsabzweigs angeordnet sein, gegebenenfalls aber auch an geeigneten Positionen zwischen dem Zulauf und dem dem Filter zugeordneten Messgerät.

Ergänzend oder alternativ zur Filterung kann die Umgehungsleitung unabhängig vom Hauptstrom aktiv durchströmt werden, beispielsweise mittels einer separat gesteuerten Pumpe. Dies ermöglicht strömungstechnisch gezielte und reproduzierbare Messbedingungen. Prinzipiell kann ein Durchströmen der Umgehungsleitung aber auch passiv bewirkt werden und/oder durch Schaltung von Ventilen. Somit können in der Umgehungsleitung / im Leitungsabzweig besonders gute Messbedingungen für apparativ aufwändige Messverfahren erzielt werden, insbesondere zur Extinktionsmessung und/oder spektroskopischen Messung der Stichproben.

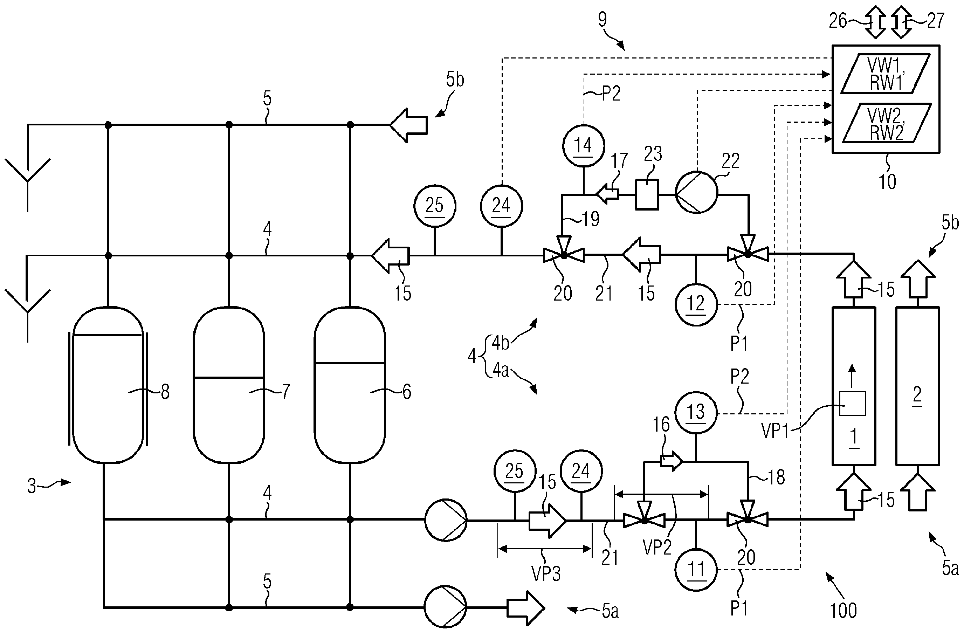

Eine bevorzugte Ausführungsform der Erfindung ist zeichnerisch dargestellt. Die einzige Figur zeigt ein Fließschema eines Reinigungssystems der Produktionsanlage.

Wie die Figur erkennen lässt, umfasst die Produktionsanlage 100 vorzugsweise wenigstens zwei Produktionseinheiten 1, 2 zur Herstellung und/oder Behandlung eines flüssigen Abfüllprodukts (nicht dargestellt), bei dem es sich beispielsweise um ein Getränk handelt.

Die Produktionsanlage 100 ist beispielsweise eine Abfüllanlage und umfasst ferner ein Reinigungssystem 3 zur ortsgebundenen Reinigung, auch Cleaning-In-Place (CIP) genannt, der Produktionseinheiten 1, 2. Bei diesen kann es sich beispielsweise um eine Kurzzeiterhitzungsanlage, eine Füllmaschine und/oder einen Pasteurisierungstunnel handeln, ebenso um eine Flaschenwaschmaschine oder dergleichen zur Behandlung von zur Abfüllung vorgesehenen Behältern.

Das Reinigungssystem 3 umfasst beispielsweise wenigstens zwei Reinigungskreisläufe 4, 5 zum Durchleiten von Reinigungsflüssigkeiten 6-8, beispielsweise einer ersten Reinigungsflüssigkeit 6 in Form einer Säure, einer zweiten Reinigungsflüssigkeit 7 in Form einer Lauge und einer dritten Reinigungsflüssigkeit 8 in Form von Heißwasser jeweils durch die Produktionseinheiten 1, 2.

Als Reinigungsflüssigkeiten 6-8 im Sinne der Erfindung sind aber auch Desinfektionslösungen, Kaltwasser / Spülwasser oder andere zur ortsgebundenen Reinigung prinzipiell geeignete flüssige Medien zu verstehen.

Einzelne Reinigungsflüssigkeiten 6-8 können auf bekannte Weise in zugeordneten Behältern, Tanks, Wärmetauschern oder dergleichen bereitgestellt und je nach Reinigungsprogramm in geeigneten Rezepturen den Reinigungskreisläufen 4, 5 über Ventile und Pumpen selektiv zugeführt werden.

Die Reinigungskreisläufe 4,5 sind vorzugsweise parallelgeschaltet, sodass die Reinigungsflüssigkeiten 6-8 vorlaufseitig auf die Reinigungskreisläufe 4,5 verteilt und rücklaufseitig wieder zusammengeführt werden können. Die Reinigungskreisläufe 4,5 sind vorzugsweise den einzelnen Produktionseinheiten 1, 2 separat zugeordnet.

Einige typische Bestandteile der Reinigungskreisläufe 4, 5 sind beispielhaft nur für den durch die Produktionseinheit 1 führenden Reinigungskreislauf 4 dargestellt und könnten in identischer oder abgewandelter Weise auch am Reinigungskreislauf 5 und/oder gegebenenfalls an weiteren Reinigungskreisläufen (nicht dargestellt) des Reinigungssystems 3 vorhanden sein.

Jeder Reinigungskreislauf 4, 5 umfasst stromaufwärts der zugeordneten Produktionseinheit 1, 2 einen Vorlauf 4a, 5a zur vorlaufseitigen Führung der Reinigungsflüssigkeiten 6-8 und stromabwärts der Produktionseinheit 1, 2 einen Rücklauf 4b, 5b zur rücklaufseitigen Führung der Reinigungsflüssigkeiten 6-8.

Das Reinigungssystem 3 umfasst ferner ein Überwachungssystem 9 mit einer elektronischen Auswerteeinheit 10 und Messgeräten 11 bis 14 zur Vergleichsmessung von wenigstens zwei für die Zusammensetzung der Reinigungsflüssigkeit 6, 7, 8 charakteristischen Parametern P1, P2.

Ein erster für die Zusammensetzung der Reinigungsflüssigkeit 6-8 charakteristischer Parameter P1 kann im Hauptstrom 15 der Reinigungsflüssigkeit 6-8 sowohl vorlaufseitig als auch rücklaufseitig kontinuierlich gemessen werden. Hierfür ist im Vorlauf 4a, 5a je ein zur prinzipiell durchgehenden Überwachung ausgebildetes erstes Messgerät 11 angeordnet, im Rücklauf 4b, 5b je ein entsprechend ausgebildetes zweites Messgerät 12.

Eine kontinuierliche Überwachung im Sinne der vorliegenden Erfindung ist gegeben, da Messfühler (nicht dargestellt) des ersten und zweiten Messgeräts 11, 12 dem Hauptstrom 15 ständig ausgesetzt werden können.

Ein zweiter für die Zusammensetzung der Reinigungsflüssigkeit 6-8 charakteristischer Parameter P2 kann sowohl an einer vorlaufseitig aus dem Hauptstrom 15 abgezweigten Stichprobe 16 von einem zur diskontinuierlichen Überwachung ausgebildeten dritten Messgerät 13 als auch an einer rücklaufseitig vom Hauptstrom 15 abgezweigten Stichprobe 17 von einem entsprechend ausgebildeten vierten Messgerät 14 gemessen werden. Somit kann der zweite charakteristische Parameter P2 an den Stichproben 16, 17 jeweils strömungstechnisch unbeeinflusst vom Hauptstrom 15 gemessen werden.

Eine diskontinuierliche Überwachung im Sinne der vorliegenden Erfindung ist gegeben, da Messfühler und/oder Messkammern (nicht dargestellt) des dritten und vierten Messgeräts 13 dem Hauptstrom 15 nicht direkt ausgesetzt sind, sondern daraus zeitlich begrenzt abgezweigten Stichproben 16, 17.

Das dritte Messgerät 13 ist in einer vom Hauptstrom abzweigenden vorlaufseitigen Umgehungsleitung 18 angeordnet, das vierte Messgerät 14 in einer vom Hauptstrom 15 abzweigenden rücklaufseitigen Umgehungsleitung 19.

Die Umgehungsleitungen 18, 19 können beispielsweise mittels elektronisch gesteuerter Dreiwegeventile 20 vorzugsweise sowohl eingangsseitig als auch ausgangsseitig bezüglich einer den Hauptstrom 15 führenden Hauptleitung 21 für die Reinigungsflüssigkeit 6-8 gezielt geöffnet und geschlossen werden. Alternativ zu den gesteuerten Dreiwegeventilen 20 können aus dem Stand der Technik bekannte Ventilschaltungen vorgesehen sein, welche insbesondere stufenlos zwischen einer offenen und geschlossenen Stellung geschaltet werden können. Somit ist es möglich, beliebig Teilmengen durch die Umgehungsleitungen 18 und 19 zu leiten.

Die Umgehungsleitungen 18, 19 können passiv durch geeignetes Öffnen der Dreiwegeventile 20 mittels des Hauptstroms 15 durchströmt (beispielhaft vorlaufseitig dargestellt) und/oder mittels einer separat steuerbaren Pumpe 22 auch unabhängig vom Hauptstrom 15 aktiv durchströmt werden (beispielhaft rücklaufseitig dargestellt).

Durch Abtrennen des jeweiligen Umgehungskreislaufs 18, 19 vom Hauptstrom 15 kann die zuvor daraus abgezweigte Stichprobe 16, 17 beispielsweise strömungslos in den Bereichen des dritten und vierten Messgeräts 13, 14 zur Bestimmung des charakteristischen Parameters P2 gemessen werden. Mit Hilfe je einer separat steuerbaren Pumpe 22 könnten die abgezweigten Stichproben 16, 17 optional bei vergleichsweise geringer, gleichmäßiger und/oder einstellbarer Strömung in den Bereichen des dritten und vierten Messgeräts 13, 14 gemessen werden.

Optional kann in wenigstens einem der Umgehungskreisläufe 18, 19 ein dem dritten und/oder vierten Messgerät 13, 14 vorgeschalteter Filter 23 angeordnet sein.

Die dargestellten Umgehungskreisläufe 18, 19 mit den zugehörigen Messgeräten 13, 14, mit der separat steuerbaren Pumpe 22 und mit dem vorgeschalteten Filter 23 sind hierbei lediglich als Beispiele für mögliche Konfigurationen einer diskontinuierlichen Überwachung des zweiten charakteristischen Parameters P2 an Stichproben 16, 17 zu verstehen.

Es könnten auch mehrere Messgeräte zur diskontinuierlichen Messung unterschiedlicher für die Zusammensetzung der Reinigungsflüssigkeit 6-8 jeweils charakteristischer (zweiter) Parameter gegebenenfalls mit separat zugeordneten Filtern 23 in wenigstens einem der Umgehungskreisläufe 18, 19 angeordnet sein.

Auch könnten mehrere Messgeräte zur kontinuierlichen Messung von für die Zusammensetzung der Reinigungsflüssigkeit 6-8 charakteristischen (ersten) Parametern im Hauptstrom 15 an der Hauptleitung 21 ausgebildet sein.

Entscheidend ist hierbei, dass die kontinuierliche Messung von für die Zusammensetzung der Reinigungsflüssigkeit 6-8 charakteristischen ersten Parametern im Hauptstrom 15 und die diskontinuierliche Messung von für die Zusammensetzung der Reinigungsflüssigkeit 6-8 charakteristischen zweiten Parametern in Stichproben 16, 17 jeweils bestimmten Volumenpaketen der durchgeleiteten Reinigungsflüssigkeit 6-8 zugeordnet sind.

Dies ist beispielhaft anhand von drei durch den ersten Reinigungskreislauf 4 strömenden (und lediglich schematisch angedeuteten) Volumenpaketen VP1-VP3 dargestellt. Demnach befindet sich ein erstes (vorderes) Volumenpaket VP1 gerade im Bereich der Produktionseinheit 1, ein zweites (mittleres) Volumenpaket VP2 im Bereich der vorlaufseitigen Umgehungsleitung 18 und ein drittes (hinteres) Volumenpaket VP 3 in einem Bereich des Vorlaufs 4a stromaufwärts davon.

Die Zuordnung einzelner Volumenpakete VP1-VP3 basiert darauf, dass deren Verweilzeiten im Reinigungskreislauf 4, 5 zwischen vorlaufseitigen und zugehörigen rücklaufseitigen Messungen berechnet werden können. Folglich lassen sich die charakteristischen Parameter P1, P2 sowohl vorlaufseitig als auch rücklaufseitig beispielsweise an ein und demselben Volumenpaket VP1, VP2 und/oder VP3 messen und gegebenenfalls im Sinne einer Differenzmessung miteinander vergleichen, insbesondere jeweils paarweise.

Paarweise Differenzmessungen am jeweils selben Volumenpaket VP1-VP3 sind insbesondere bei der kontinuierlichen Messung des ersten charakteristischen Parameters P1 im Hauptstrom 15 auf einfache Weise durch Auswahl zeitlich zueinander passender Messwerte des ersten und zweiten Messgeräts 11, 12 möglich.

Je nach Messzyklen und Messdauern der diskontinuierlich arbeitenden Messgeräte 13, 14 in den Umgehungsleitungen 18, 19 ist eine Zuordnung von Volumenpaketen VP1-VP3 und jeweiligen vorlaufseitigen und rücklaufseitigen Messungen des zweiten charakteristischen Parameters P2 durch zeitliche passende Terminierung der einzelnen Messungen möglich, ersatzweise aber auch durch Interpolation aus vorangegangenen und nachfolgenden Messungen beispielsweise wie folgt denkbar.

In der Regel kann davon ausgegangen werden, dass man vorlaufseitig beide charakteristischen Parameter P1, P2 direkt an jedem der Volumenpakte VP1-VP3 bzw. an einer daraus abgezweigten Stichprobe 16 messen kann und somit für jedes Volumenpaket VP1-VP3 je einen ersten Vorlaufwert VW1 des ersten charakteristischen Parameters P1 und je einen zweiten Vorlaufwert VW2 des zweiten charakteristischen Parameters P2 erhält. Dies liegt daran, dass man im Vorlauf 4a normalerweise zu beliebigen Zeitpunkten Stichproben 16 aus dem Hauptstrom 15 abzweigen und/oder Messungen durchführen kann. Es ist dann lediglich das jeweils dafür verwendete Volumenpaket VP1-VP3 zuzuordnen und durch den Reinigungskreislauf 4, 5 nachzuverfolgen.

Ferner kann davon ausgegangen werden, dass auch die rücklaufseitige Messung des ersten charakteristischen Parameters P1 aufgrund der prinzipiell kontinuierlichen Überwachung stets direkt an jedem der Volumenpakete VP1-VP3 möglich ist und man daraus jeweils einen ersten Rücklaufwert RW1 des ersten charakteristischen Parameters P1 erhält. Hierzu muss die jeweilige Messung lediglich zu einem Zeitpunkt erfolgen, an dem das zugehörige Volumenpaket VP1-VP3 durch den Bereich des dritten Messgeräts 13 strömt.

Demgegenüber wäre es aber denkbar, dass das vierte Messgerät 14 zu einem Zeitpunkt, an dem eine rücklaufseitige Stichprobe 17 beispielsweise aus dem anströmenden zweiten Volumenpaket VP2 abgezweigt werden müsste, aufgrund eines bestimmten Ablaufs von Messzyklen noch nicht wieder für die diskontinuierliche Messung verfügbar ist. Es könnte dann sein, dass die rücklaufseitige Stichprobe 17 aus dem vorbeiströmenden zweiten Volumenpaket VP2 nicht abgezweigt werden kann und an diesem folglich kein zugehöriger Rücklaufwert RW2 des zweiten Parameters P2 gemessen werden kann. Aus diesem Grund zunächst fehlende Rücklaufwerte RW2 einzelner Volumenpakete (beispielsweise VP2) könnten dann beispielsweise durch Interpolation aus den Rücklaufwerten RW2 vorauseilender und nachfolgender Volumenpakete (beispielsweise VP1 und VP3) ersatzweise berechnet werden.

Ebenso wäre es denkbar, eine ausgefallene rücklaufseitige Messung des zweiten charakteristischen Parameters P2 nachzuholen, sobald das vierte Messgerät 14 wieder verfügbar ist und/oder eine Stichprobe 17 abgezweigt werden kann. In diesem Fall könnte ein Rücklaufwert RW2 für ein Ersatz-Volumenpaket (nicht dargestellt) gemessen werden. Ein zugehöriger Vorlaufwert VW2 für das Ersatz-Volumenpaket könnte dann durch Interpolation und/oder Extrapolation bereits zuvor gemessener Vorlaufwerte VW2 von dem Ersatz-Volumenpaket vorausgeeilten und/oder nachgefolgten Volumenpaketen berechnet werden.

Vorlaufwerte VW1 und zugehörige Rücklaufwerte RW1 des ersten charakteristischen Parameters P1 können aufgrund seiner kontinuierlichen Überwachung den Volumenpaketen VP1-VP3 in der Regel direkt zugeordnet werden. Eine entsprechende direkte Zuordnung zu den Vorlaufwerten VW2 und zugehörigen Rücklaufwerten RW2 des diskontinuierlich gemessenen zweiten charakteristischen Parameters P2 ist prinzipiell vorteilhaft, kann aber hilfsweise durch Interpolation und/oder Extrapolation von Vorlaufwerten VW2 und/oder Rücklaufwerten RW2 vorauseilender und/oder nachfolgender Volumenpakete ersetzt werden.

Eine zeitliche und örtliche Nachverfolgung und/oder Bestimmung von Verweildauern einzelner Volumenpakete VP1-VP3 der Reinigungsflüssigkeit 6-8 zwischen den vorlaufseitigen und rücklaufseitigen Messungen der charakteristischen Parameter P1, P2 ist durch wenigstens eine Durchflussmessung im Hauptstrom 15 mittels eines Durchflussmessers 24 unter Berücksichtigung des Fassungsvermögens des jeweiligen Reinigungskreislaufs 4, 5 einschließlich der jeweils von der Reinigungsflüssigkeit 6-8 durchströmten Abschnitte der Produktionseinheit 1, 2 gegeben.

Das Fassungsvermögen von Produktionseinheiten 1, 2 ergibt sich aus den Volumina ihrer bei der Reinigung durchströmten Leitungen und Behälter und bei deren nicht vollständiger Füllung gegebenenfalls durch zugehörige Füllstände. Diese Werte können bauseitig vorgegeben sein oder, insbesondere im Fall von Füllständen, während der Reinigung überwacht werden und dann jeweils in die Berechnung der für die Nachverfolgung einzelner Volumenpakete VP1-VP3 anzusetzenden Kreislaufvolumina eingehen.

Sind durchströmte Volumina und der Durchfluss bekannt, so lassen sich die Verweildauern der einzelnen Volumenpakete VP1-VP3 zwischen den jeweiligen Messgeräten 11-14 berechnen und für die gegenseitige Zuordnung der Vorlaufwerte VW1, VW2 und Rücklaufwerte RW1, RW2 einzelner Volumenpakete VP1-VP3 in der Auswerteeinheit 10 verwenden.

Beispielhaft dargestellt sind vorlaufseitig und rücklaufseitig im Reinigungskreislauf 4 angeordnete Temperaturmesser 25, die beispielsweise der Prozessüberwachung der ortsgebundenen Reinigung dienen und ebenso wie die Durchflussmesser Messwerte von Parametern liefern, die nicht im Sinne der vorliegenden Erfindung für die Zusammensetzung der Reinigungsflüssigkeit 6-8 charakteristisch sind, sondern Prozessparameter der ortsgebundenen Reinigung betreffen.

Der Übersichtlichkeit halber ist teilweise lediglich für den Rücklauf 4b und stellvertretend für den Rücklauf 5b und die Vorläufe 4a, 5a dargestellt, dass die Messgeräte 11 bis 14, Durchflussmesser 24 und optional separat steuerbare Pumpen 22 mit der Auswerteeinheit 10 kommunizieren, um darin die Vorlaufwerte VW1, VW2 und zugehörigen Rücklaufwerte RW1, RW2 einzelnen Volumenpaketen VP1-VP3 zuzuordnen und schließlich maschinell miteinander zu vergleichen.

Der Vergleich derart einander zugeordneter Vorlaufwerte VW1 und Rücklaufwerte RW1 sowie Vorlaufwerte VW2 und Rücklaufwerte RW2 kann im einfachsten Fall als reine Differenzmessung verstanden werden. Prinzipiell sind aber beliebige Berechnungsalgorithmen zum Vergleich einander zugeordneter Vorlaufwerte VW1, VW2 und Rücklaufwerte RW1, RW2 denkbar, gegebenenfalls auch unter Berücksichtigung einer statistischen Auswertung.

Prinzipiell sollten die jeweils (paarweise) miteinander zu vergleichende Vorlaufwerte VW1, VW2 und Rücklaufwerte RW1, RW2 möglichst demselben Volumenpaket VP1-VP3 zugeordnet werden, um einen möglichst präzisen Vergleich des vorlaufseitigen und rücklaufseitigen Zustands der jeweiligen Reinigungsflüssigkeit 6-8 zu ermöglichen. Ein derartiger Zustandsvergleich dient dann dazu, den Reinigungsfortschritt eines bestimmten Reinigungsprogramms (Steuerungsprogramms) 26 zu überwachen und gegebenenfalls bestimmte Prozessschritte des Reinigungsprogramms 26 zielgerichtet zu beenden oder einzuleiten.

Ändert sich beispielsweise ein festgestellter Unterschied zwischen Vorlaufwerten VW1, VW2 und zugeordneten Rücklaufwerten RW1, RW2 eines bestimmten charakteristischen Parameters P1, P2 nicht mehr signifikant, so kann von einer abgeschlossenen Reinigung oder einer im weiteren Verlauf nicht mehr wirksamen Reinigung ausgegangen werden und der jeweilige Prozessschritt beendet, unterbrochen oder nach Austausch der Reinigungsflüssigkeit 6-8 fortgesetzt werden.

Ebenso könnten bestimmte Schwellenwerte für die einzelnen charakteristischen Parameter P1, P2 vorgegeben werden und bei deren Erreichen ein bestimmter Prozessschritt automatisch beendet oder eine entsprechende Meldung ausgegeben werden. Beispielsweise könnte es sich bei den Schwellenwerten um vorgegebene Rücklaufwerte RW1, RW2 handeln oder um vorgegebene Differenzwerte aus einander paarweise zugeordneten Vorlaufwerten VW1, VW2 und Rücklaufwerten RW1, RW2.

Bei der kontinuierlichen Messung im Hauptstrom 15 kann es sich beispielsweise um eine Leitwertmessung, Trübungsmessung und/oder pH-Wertmessung handeln. Es versteht sich hierbei, dass dann mehrere unterschiedliche Messgeräte 11, 12 sowohl vorlaufseitig als auch rücklaufseitig für die einzelnen zu bestimmenden charakteristischen Parameter P1 vorhanden sind.

Die Messgeräte 13, 14 in den Umgehungsleitungen 18, 19 sind vorzugsweise optische Messgeräte, mit denen sich eine Extinktion beispielsweise durch Abschwächung von Infrarotlicht und/oder in einem bestimmten Spektralbereich oder bei unterschiedlichen Wellenlängen bestimmen lässt. Dadurch lassen sich Wirkstoffe oder andere Inhaltsstoffe der Reinigungsflüssigkeiten 6-8 gegebenenfalls sowohl qualitativ als auch quantitativ bestimmen. Solche Messungen sind in den Umgehungsleitungen 18, 19 aus strömungstechnischer Sicht unter reproduzierbaren und gegebenenfalls gezielt veränderbaren Bedingungen möglich. Zudem können die für derartige Messung erforderlichen Messdauern und Messzyklen durch Entnahme von Stichproben 16, 17 gezielt und flexibel angepasst werden. Dies ermöglicht beispielsweise bessere Signal-Rausch-Verhältnisse als bei entsprechender kontinuierlicher Messung im Hauptstrom 15.

Die Präzision und Aussagekraft von Messungen für die Zusammensetzung der Reinigungsflüssigkeit 6-8 charakteristischer Parameter P1, P2 lässt sich sowohl kontinuierlich im Hauptstrom als auch diskontinuierlich in Stichproben 16, 17 durch die Zuordnung der derart gewonnenen Vorlaufwerte VW1, VW2 und Rücklaufwerte RW1, RW2 zu einzelnen Volumenpaketen VP1-VP3 gegenüber einer paarweise zeitgleichen vorlaufseitigen und rücklaufseitigen Messung prinzipiell verbessern und gegebenenfalls an unterschiedliche Reinigungsprogramme 26 gezielt anpassen.

Vorteilhaft ist zudem, dass entsprechende Messungen und Auswertungen bei Parallelschaltung von Reinigungskreisläufen 4, 5 in diesen separat unter entsprechender Zuordnung von Vorlaufwerten VW1, VW2 und Rücklaufwerten RW1, RW2 mit Volumenpaketen VP1-VP3 möglich sind.

Die Auswerteeinheit 10 kann für die Auswertung der Vorlaufwerte VW1, VW2 und Rücklaufwerte RW1, RW2 prinzipiell bekannte Algorithmen verwenden und erzielt die verbesserte technische Wirkung im Wesentlichen durch eine vorlauf-/rücklaufseitige Vergleichsmessung, insbesondere Differenzmessung, an einander zugeordneten und insbesondere identischen Volumenpaketen VP1-VP3.

Ferner ermöglicht die Auswerteeinheit 10 durch geeignete Programmierung eine Optimierung von Reinigungsprogrammen (Steuerungsprogrammen) 26 und Produktionsprogrammen (Steuerungsprogrammen) 27 unter gegebenenfalls statistischer und/oder historischer Auswertung der auf die beschriebene Weise ermittelten Vorlaufwerte VW1, VW2 und Rücklaufwerte RW1, RW2. Hierbei können weitere für die ortsgebundene Reinigung relevante Maschinenparameter und Prozessparameter, wie beispielsweise Durchflusswerte, Medientemperaturen, Medienverbräuche, Kreislaufzeiten für die einzelnen Reinigungsflüssigkeiten 6-8 oder dergleichen in die Auswertung einfließen und gegebenenfalls unter Zuhilfenahme selbstlernender Algorithmen (künstliche Intelligenz) auch automatisch optimiert werden.

Hierbei können auch für die jeweilige Produktionsanlage 100 und die darin vorhandenen Produktionseinheiten 1, 2 charakteristische Daten, wie beispielsweise Maschinenidentifikation, und für einzelne Reinigungsprogramme und Produktionsprogramme relevante Daten einbezogen werden. Auch derartige Auswertungen werden insgesamt durch die auf einzelne Volumenpakete bezogene Auswertung von Vorlaufwerten VW1, VW2 und Rücklaufwerten RW1, RW2 verbessert.

## Patentansprüche

1. Verfahren zur Prozessüberwachung einer ortsgebundenen Reinigung wenigstens einer Produktionseinheit (1, 2) zur Herstellung und/oder Behandlung flüssiger Abfüllprodukte, insbesondere Getränke, wobei eine Reinigungsflüssigkeit (6-8) durch einen durch die Produktionseinheit führenden Reinigungskreislauf (4, 5) geleitet wird und dabei wenigstens ein für die Zusammensetzung der Reinigungsflüssigkeit charakteristischer Parameter (P1, P2) im Vorlauf (4a, 5a) und Rücklauf (4b, 5b) des Reinigungskreislaufs im Vergleich zueinander gemessen wird, wobei dabei ermittelte Vorlaufwerte (VW1, VW2) und Rücklaufwerte (RW1, RW2) des charakteristischen Parameters durch den Reinigungskreislauf strömenden Volumenpaketen (VP1-VP3) der Reinigungsflüssigkeit insbesondere paarweise maschinell zugeordnet werden und ein Fortschritt der Reinigung und/oder eine dadurch bedingte Veränderung der Reinigungsflüssigkeit durch Vergleich der den Volumenpaketen jeweils zugeordneten Vorlaufwerte und Rücklaufwerte maschinell beurteilt wird, wobei Verweildauern einzelner Volumenpakete (VP1-VP3) zwischen vorlaufseitigen und zugehörigen rücklaufseitigen Messungen sowohl unter Berücksichtigung des Fassungsvermögens dazwischenliegender Abschnitte des Reinigungskreislaufs (4, 5) und/oder zugehöriger Füllstände als auch auf der Grundlage einer Volumenstrommessung im Hauptstrom (15) der Reinigungsflüssigkeit (6-8) ermittelt werden.

2. Verfahren nach Anspruch 1, wobei wenigstens ein erster für die Zusammensetzung der Reinigungsflüssigkeit (6-8) charakteristischer Parameter (P1) durch kontinuierliche Überwachung in einem Hauptstrom (15) der Reinigungsflüssigkeit (15) am jeweiligen Volumenpaket (V1-V3) gemessen wird und insbesondere eine Leitfähigkeit, eine Trübung oder ein pH-Wert ist.

3. Verfahren nach Anspruch 1 oder 2, wobei wenigstens ein für die Zusammensetzung der Reinigungsflüssigkeit (6-8) charakteristischer zweiter Parameter (P2) an einer im jeweiligen Volumenpaket (VP1-VP3) enthaltenen Stichprobe (16, 17) insbesondere mittels spektroskopischer Messung und/oder mittels Infrarot-Extinktionsmessung ermittelt wird und die Stichprobe dafür aus einem Hauptstrom (15) der Reinigungsflüssigkeit abgezweigt wird.

4. Verfahren nach Anspruch 3, wobei die abgezweigte Stichprobe (16, 17) unabhängig vom Hauptstrom (15) in den Bereich der zugeordneten Messung gefördert und/oder vor der Messung gefiltert und/oder bei der Messung vorübergehend vom Hauptstrom abgetrennt und/oder nach der Messung wieder in den Hauptstrom zurückgeführt wird.

5. Verfahren nach wenigstens einem der vorigen Ansprüche, wobei die Reinigungsflüssigkeit (6-8) durch parallelgeschaltete Reinigungskreisläufe (4, 5) geleitet wird und für diese separat jeweils Vorlaufwerte (VW1, VW2) und Rücklaufwerte (RW1, RW2) ermittelt, hindurchgeleiteten Volumenpaketen (VP1-VP3) insbesondere paarweise zugeordnet und miteinander verglichen werden.

6. Verfahren nach wenigstens einem der vorigen Ansprüche, wobei für den Rücklaufwert (RW1, RW2) ein absoluter oder ein auf den Vorlaufwert (VW1, VW2) bezogener relativer Schwellenwert vorgegeben und durch dessen Erreichen ein zugeordneter Reinigungsfortschritt maschinell festgestellt wird.

7. Verfahren nach wenigstens einem der vorigen Ansprüche, wobei ein Steuerungsprogramm (26) für die ortsgebundene Reinigung und/oder ein Steuerungsprogramm (27) für einen Produktionsbetrieb der Produktionseinheit (1, 2) mittels eines Vergleichs der jeweils einander zugeordneten Vorlaufwerte (VW1, VW2) und Rücklaufwerte (RW1, RW2) für mehrere Volumenpakete (VP1-VP3) automatisch angepasst wird.

8. Verfahren nach wenigstens einem der vorigen Ansprüche, wobei die Vorlaufwerte (VW1, VW2) und Rücklaufwerte (RW1, RW2) ferner einem bei der ortsgebundenen Reinigung der Produktionseinheit (1, 2) abgelaufenen Steuerungsprogramm (26) und wenigstens einem der folgenden dabei verwendeten Reinigungsparameter maschinell zugeordnet werden: Typen von Reinigungsflüssigkeiten; Grenzwerte für Leitwert und/oder Temperatur; Volumenströme der Reinigungsflüssigkeiten; Reinigungsdauer; Störungsmeldungen; Zeitstempel für Reinigungsbeginn/ -ende; und Medienverbräuche.

9. Verfahren nach Anspruch 8, wobei wenigstens zwei der Reinigungsparameter und/oder die Vorlauf- und Rücklaufwerte (VW1, VW2, RW1, RW2) spezifisch für die Produktionseinheit (1, 2) und das abgelaufene Steuerungsprogramm (26) historisch miteinander verglichen werden und in eine maschinelle Analyse eines Heizverhaltens der Produktionseinheit, der Medienverbräuche der Reinigung, der Konzentrationen von Reinigungsflüssigkeiten (6-8) und/oder der Zeiten für deren Kreislauf und Wechsel einfließt.

10. Produktionsanlage (100) mit wenigstens einer Produktionseinheit (1, 2) zur Herstellung und/oder Behandlung flüssiger Abfüllprodukte, insbesondere Getränke, und mit einem Reinigungssystem (3) zur ortsgebundenen Reinigung der Produktionseinheit, wobei das Reinigungssystem wenigstens einen durch die Produktionseinheit führenden Reinigungskreislauf (4, 5) zum Durchleiten einer Reinigungsflüssigkeit (6-8) und ein Überwachungssystem (9) mit einer Auswerteeinheit (10) und zugeordneten Messgeräten (11-14) zur Vergleichsmessung wenigstens eines für die Zusammensetzung der Reinigungsflüssigkeit charakteristischen Parameters (P1, P2) sowohl im Vorlauf (4a, 5a) als auch im Rücklauf (4b, 5b) des Reinigungskreislaufs umfasst, wobei im Reinigungskreislauf vorlaufseitige und/oder rücklaufseitige Durchflussmesser (24) angeordnet sind und die Auswerteeinheit eingerichtet ist, mit den Messgeräten ermittelte Vorlaufwerte (VW1, VW2) und Rücklaufwerte (RW1, RW2) des charakteristischen Parameters wenigstens einem durch den Reinigungskreislauf strömenden Volumenpaket (VP1-VP3) der Reinigungsflüssigkeit insbesondere paarweise zuzuordnen und einen Fortschritt der Reinigung und/oder eine dadurch bedingte Veränderung der Reinigungsflüssigkeit durch Vergleich der Vorlaufwerte und Rücklaufwerte des Volumenpakets zu beurteilen, und wobei die Auswerteeinheit ferner eingerichtet ist, Verweildauern einzelner Volumenpakete (VP1-VP3) zwischen vorlaufseitigen und zugehörigen rücklaufseitigen Messungen sowohl unter Berücksichtigung des Fassungsvermögens dazwischenliegender Abschnitte des Reinigungskreislaufs (4, 5) und/oder zugehöriger Füllstände als auch auf der Grundlage einer Volumenstrommessung im Hauptstrom (15) der Reinigungsflüssigkeit (6-8) zu ermitteln.

11. Produktionsanlage nach Anspruch 10, wobei wenigstens eines der Messgeräte (11, 12) zur kontinuierlichen Überwachung einer Leitfähigkeit, einer Trübung und/oder eines pH-Werts im Hauptstrom (15) der Reinigungsflüssigkeit ausgebildet ist.

12. Produktionsanlage nach Anspruch 11 oder 10, wobei wenigstens eines der Messgeräte (13, 14) in einer einen Hauptstrom (15) der Reinigungsflüssigkeit (6-8) umgehenden Umgehungsleitung (18, 19) zum Abzweigen einer Stichprobe (16, 17) aus der Reinigungsflüssigkeit angeordnet und zur qualitativen und/oder quantitativen Bestimmung von Bestandteilen der Reinigungsflüssigkeit insbesondere mittels spektroskopischer Messung und/oder Infrarot-Extinktionsmessung ausgebildet ist.

13. Produktionsanlage nach Anspruch 12, wobei in der Umgehungsleitung (19) eine Pumpe (22) zum Fördern der abgezweigten Stichprobe (17) zum Messgerät (14) und/oder ein dem Messgerät vorgeschalteter Filter (23) angeordnet ist, und/oder mit wenigstens einem Ventil (20) zum Abtrennen der Umgehungsleitung vom Hauptstrom.

## Claims

1. Method for process monitoring stationary cleaning of at least one production unit (1, 2) for making and/or treating liquid products to be filled, in particular beverages, wherein a cleaning liquid (6-8) is lead through a cleaning circuit (4, 5) extending through the production unit while measuring a parameter (P1, P2) characteristic of the composition of the cleaning liquid in the advance (4a, 5a) and the return (4b, 5b) of the cleaning circuit in comparison with each other, wherein measured advance values (VW1, VW2) and return values (RW1, RW2) of the characteristic parameter are allocated, in particular in pairs, volume packets (VP1-VP3) of the cleaning liquid flowing through the cleaning circuit by machine and a progress of the cleaning process and/or a variation of the cleaning liquid caused thereby is evaluated by machine through comparison of the forward values and return values respectively allocated to the volume packets, wherein dwell times of individual volume packets (VP1-VP3) between advance-side and return-side measurements are determined by taking into account both the capacity of sections of the cleaning circuit (4, 5) therebetween and/or filling levels associated therewith as well as based on a volume flow measurement in the main stream (15) of the cleaning liquid (6-8).

2. Method according to claim 1, wherein at least a first of the parameters (P1) characteristic of the composition of the cleaning liquid (6-8) is measured by continuously monitoring a main stream (15) of the cleaning liquid (15) at the respective volume packet (V1-V3) being, in particular, a conductance, a turbidity or a pH value.

3. Method according to claims 1 or 2, wherein at least a second parameter (P2) characteristic of the composition of the cleaning liquid (6-8) is determined from a sample (16, 17) contained in the respective volume packet (VP1-VP3), in particular by means of spectroscopic measurement and/or by means of infrared extinction measurement and wherein the sample is taken from a mainstream (15) of the cleaning liquid.

4. Method according to claim 3, wherein the sample (16, 19) taken is fed to the region of the allocated measurement independently of the main stream (15) and/or filtered prior to measurement and/or separated from the main stream during measurement and/or returned to the main stream after measurement.

5. Method according to any one of the preceding claims, wherein the cleaning liquid (6-8) is lead via parallel cleaning circuits (4, 5) and respective advance values (VW1, VW2) and return values (RW1, RW2) are determined therefore separately, and are allocated to volume packets (VP1-VP3) passed through, in particular in pairs, and compared to each other.

6. Method according to any one of the preceding claims, wherein for the return value (RW1, RW2) an absolute threshold value or a relative threshold value related to the advance value (VW1, VW2) is preset and, when reaching the same, an associated cleaning progress is established by machine.

7. Method according to any one of the preceding claims, wherein a control program (26) for the stationary cleaning and/or a control program for a production process of the production unit (1, 2) are automatically adjusted by means of a comparison of the advance values (VW1, VW2) and the return values (RW1, RW2) associated with each other for a plurality of volume packets (VP1-VP3).

8. Method according to any one of the preceding claims, wherein the advance values (VW1, VW2) and the return values (RW1, RW2) are further allocated by machine to a control program (26) expired for the stationary cleaning of the production unit (1, 2) and at least one of the following cleaning parameters used therein: types of cleaning liquids; thresholds for conductance and/or temperature; volume flows of the cleaning liquids; duration of cleaning process; fault signal; timestamp for beginning/end of cleaning process and usage of media.

9. Method according to claim 8, wherein histories of at least two of the parameters and/or the advance and return values (VW1, VW2, RW1, RW2) specific for the production unit (1, 2) and the expired control program (26) are compared to each other and the media usage in the cleaning process, the concentrations of cleaning liquids (6-8) and/or the timing of circulation and exchange thereof are included in a machine analysis of a heating behaviour of the production unit.

10. Production plant (100) comprising at least one production unit (1, 2) for making and/or treating liquid products to be filled, in particular beverages, and a cleaning system (3) for stationary cleaning of the production unit, wherein the cleaning system comprises at least one cleaning circuit (4, 5) extending through the production unit for leading a cleaning liquid (6-8) and a monitoring system (9) including an evaluation unit (10) and associated measuring devices (11-14) for comparative measurement of at least one parameter (P1, P2) characteristic of the composition of the cleaning liquid both in the advance (4a, 5a) and in the return (4b, 5b) of the cleaning circuit wherein in the cleaning circuit advance-side and/or return-side flow meters (24) are provided and the evaluation unit is configured to allocate advance values (VW1, VW2) and return values (RW1, RW2) of the characteristic parameter detected by the measuring devices to at least one volume packet (VP1-VP3) flowing through the cleaning circuit, in particular in pairs, and to evaluate a progress of the cleaning process and/or a variation of the cleaning liquid caused thereby by comparing the advance values and the return values of the volume packet and wherein the evaluation unit is further configured to determine dwell times of individual volume packets (VP1-VP3) between advance-side and return-side measurements by taking into account both the capacity of sections of the cleaning circuit (4, 5) therebetween and/or filling levels associated therewith as well as based on a volume flow measurement in the main stream (15) of the cleaning liquid (6-8).

11. Production plant according to claim 10, wherein at least one of the measuring devices (11, 12) is configured for continuously monitoring a conductance, a turbidity and/or a pH value of the main stream (15) of the cleaning liquid.

12. Production plant according to claims 11 or 10, wherein at least one of the measuring devices (13, 14) is arranged in a bypass line (18, 19) bypassing the main stream (15) of the cleaning liquid (6-8) for taking a sample (16, 17) from the cleaning liquid and/or for qualitatively and/or quantitavely determining components of the cleaning liquid, in particular by means of spectroscopic measurement and/or by means of infrared extinction measurement.

13. Production plant according to claim 12, wherein in the bypass line (19) a pump (22) for feeding the sample (17) taken to the measuring device (14) and/or a filter (23) upstream of the measuring device are arranged, and/or comprising at least a valve (20) for disconnecting the bypass line from the main stream.

## Revendications

1. Procédé de surveillance d'une opération de nettoyage localisé d'au moins une unité de production (1, 2) destinée à la fabrication et/ou au traitement de produits liquides à mettre en bouteille, en particulier de boissons, dans lequel un liquide de nettoyage (6-8) est guidé à travers un circuit de nettoyage (4, 5) traversant l'unité de production et au moins un paramètre (P1, P2) caractéristique de la composition du liquide de nettoyage au sein du flux aller (4a, 5a) et du flux retour (4b, 5b) du circuit de nettoyage est alors mesuré avec comparaison de l'un et de l'autre, dans lequel des valeurs de flux aller (VW1, VW2) et des valeurs de flux retour (RW1, RW2), déterminées à cette occasion, du paramètre caractéristique sont associées de manière automatique, en particulier par paires, à des portions volumiques (VP1-VP3) de liquide de nettoyage traversant le circuit de nettoyage et la progression du nettoyage et/ou une modification résultante du liquide de nettoyage est/sont évaluée(s) de manière automatique par comparaison des valeurs de flux aller et des valeurs de flux retour respectivement associées aux portions volumiques, dans lequel les temps de séjour de portions volumiques (VP1-VP3) individuelles entre des mesures effectuées côté flux aller et des mesures associées effectuées côté flux retour sont déterminés à la fois en prenant en compte la capacité de contenance de sections intermédiaires du circuit de nettoyage (4, 5) et/ou les niveaux de remplissage associés et en se basant sur une mesure de débit volumique au sein du flux principal (15) du liquide de nettoyage (6-8).

2. Procédé selon la revendication 1, dans lequel au moins un premier paramètre (P1) caractéristique de la composition du liquide de nettoyage (6-8) est mesuré par surveillance continue au sein d'un flux principal (15) du liquide de nettoyage (15) au niveau d'une portion volumique (V1-V3) respective et est en particulier une conductivité, une turbidité ou une valeur de pH.

3. Procédé selon la revendication 1 ou 2, dans lequel au moins un deuxième paramètre (P2) caractéristique de la composition du liquide de nettoyage (6-8) est déterminé au niveau d'un échantillon (16, 17) contenu dans la portion volumique (VP1-VP3) respective, en particulier au moyen d'une mesure spectroscopique et/ou au moyen d'une mesure d'extinction de l'infrarouge et l'échantillon est pour cela détourné d'un courant principal (15) du liquide de nettoyage.

4. Procédé selon la revendication 3, dans lequel l'échantillon (16, 17) détourné est transporté indépendamment du flux principal (15) dans la région de la mesure associée et/ou est filtré avant la mesure et/ou est temporairement séparé du flux principal lors de la mesure et/ou est renvoyé à nouveau dans le flux principal après la mesure.

5. Procédé selon au moins l'une quelconque des revendications précédentes, dans lequel le liquide de nettoyage (6-8) est guidé à travers des circuits de nettoyage (4, 5) connectés en parallèle et des valeurs de flux aller (VW1, VW2) et des valeurs de flux retour. (RW1, RW2) sont respectivement déterminées de manière séparée pour chacun desdits circuits, et des portions volumiques (VP1-VP3) guidées à travers lesdits circuits sont en particulier associées par paires et comparées entre elles.

6. Procédé selon au moins l'une quelconque des revendications précédentes, dans lequel une valeur de seuil absolue ou une valeur de seuil relative liée à la valeur de flux aller (VW1, VW2) est spécifiée pour la valeur de flux retour (RW1, RW2) et une progression de nettoyage associée est déterminée de manière automatique lorsque ladite valeur de seuil est atteinte.

7. Procédé selon au moins l'une quelconque des revendications précédentes, dans lequel un programme de commande (26) destiné à un nettoyage localisé et/ou un programme de commande (27) destiné à un fonctionnement en production de l'unité de production (1, 2) est ajusté de manière automatique au moyen d'une comparaison entre les valeurs de flux aller (VW1, VW2) et les valeurs de flux retour (RW1, RW2) respectivement associées entre elles pour plusieurs portions volumiques (VP1-VP3).

8. Procédé selon au moins l'une quelconque des revendications précédentes, dans lequel les valeurs de flux aller (VW1, VW2) et les valeurs de flux retour (RW1, RW2) sont en outre associées de manière automatique à un programme de commande (26) ayant été exécuté lors du nettoyage localisé de l'unité de production (1, 2) et à au moins un des paramètres de nettoyage ci-dessous utilisés à cette occasion : types de liquides de nettoyage ; valeurs limites de conductance et/ou de température ; débits volumiques des liquides de nettoyage ; temps de nettoyage ; rapports de pannes ; horodatage de début/fin de nettoyage ; et consommations de services.

9. Procédé selon la revendication 8, dans lequel les historiques d'au moins deux des paramètres de nettoyage et/ou des valeurs de flux aller et de flux retour (VW1, VW2, RW1, RW2) sont comparés entre eux spécifiquement pour l'unité de production (1, 2) et pour le programme de commande (26) ayant été exécuté et sont intégrés dans une analyse automatique d'un comportement thermique de l'unité de production, des consommations de services du nettoyage, des concentrations de liquides de nettoyage (6-8) et/ou des temps consacrés à leur circulation et à leur remplacement.

10. Installation de production (100) comprenant au moins une unité de production (1, 2) permettant de fabriquer et/ou de traiter des produits liquides à mettre en bouteille, en particulier des boissons, et comprenant un système de nettoyage (3) permettant de nettoyer de manière localisée l'unité de production, dans laquelle le système de nettoyage comprend au moins un circuit de nettoyage (4, 5) traversant l'unité de production et permettant de guider un liquide de nettoyage (6-8) et un système de surveillance (9) comprenant une unité d'évaluation (10) et des dispositifs de mesure (11-14) associés permettant de mesurer de manière comparative au moins un paramètre (P1, P2) caractéristique de la composition du liquide de nettoyage aussi bien dans le flux aller (4a, 5a) que dans le flux retour (4b, 5b) du circuit de nettoyage, dans laquelle des débitmètres (24) situés côté flux aller et/ou côté flux retour sont agencés dans le circuit de nettoyage et l'unité d'évaluation est conçue pour associer, en particulier par paires, des valeurs de flux aller (VW1, VW2) et des valeurs de flux retour (RW1, RW2), déterminées par les appareils de mesure, du paramètre caractéristique à au moins une portion volumique (VP1-VP3) du liquide de nettoyage traversant le circuit de nettoyage, et pour évaluer la progression du nettoyage et/ou une modification résultante du liquide de nettoyage grâce à une comparaison entre les valeurs de flux aller et les valeurs de flux retour de la portion volumique, et dans laquelle l'unité d'évaluation est en outre conçue pour déterminer les temps de séjour de portions volumiques (VP1-VP3) individuelles entre des mesures situées côté flux aller et des mesures situées côté flux retour associées à la fois en prenant en compte la capacité de contenance de sections intermédiaires du circuit de nettoyage (4, 5) et/ou de niveaux de remplissage associés et en se basant sur une mesure de débit volumique au sein du flux principal (15) du liquide de nettoyage (6-8).

11. Installation de production selon la revendication 10, dans laquelle au moins un des appareils de mesure (11, 12) est conçu pour surveiller en continu une conductivité, une turbidité et/ou une valeur de pH dans le flux principal (15) du liquide de nettoyage.

12. Installation de production selon la revendication 11 ou 10, dans laquelle au moins un des appareils de mesure (13, 14) est agencé dans une conduite de dérivation (18, 19) contournant un flux principal (15) du liquide de nettoyage (6-8) et permettant de détourner un échantillon (16, 17) à partir du liquide de nettoyage et est conçu pour déterminer de manière qualitative et/ou quantitative des composants du liquide de nettoyage, en particulier au moyen d'une mesure spectroscopique et/ou d'une mesure d'extinction de l'infrarouge.

13. Installation de production selon la revendication 12, dans laquelle une pompe (22) permettant de refouler l'échantillon (17) détourné vers l'appareil de mesure (14) et/ou un filtre (23) connecté en amont de l'appareil de mesure est/sont agencé(s) dans la conduite de dérivation (19), et/ou comportant au moins une vanne (20) permettant de séparer la conduite de dérivation et le flux principal.
